# EUROPEAN PATENT SPECIFICATION

(11) **EP 1 600 061 B1**
(45) Date of publication and mention of the grant of the patent: **12.05.2010**
(21) Application number: 04012288.9
(22) Date of filing: 25.05.2004
(51) Int. Cl.: A23L 1/30, A23L 1/307, A23L 1/308, A61K 31/35, A61K 31/702, A61K 31/716, A61K 31/16, A61K 36/185, A61K 36/48, A61K 36/63, A61K 36/77, A61K 36/82, A61K 45/06, A61Q 19/00

(54) **Oral and/or topical compositions**
Orale und/oder topische Zubereitungen
Compositions buccales et/ou topiques

(43) Date of publication of application: 30.11.2005
(73) Proprietor: Cognis IP Management GmbH, 40589 Düsseldorf (DE)
(72) Inventor: Rull Prous, Santiago, 08034 Barcelona (ES); Fabry, Bernd, Dr., 41352 Korschenbroich (DE); Bell, Doris, Dr., 40627 Düsseldorf (DE)
(74) Representative: Lavé, Stéphanie

(56) References cited:
- EP-A- 0 524 484
- WO-A-00/64282
- WO-A-02/47493
- WO-A-96/03150
- WO-A-03/013438
- WO-A-03/077671
- US-A- 5 071 653
- US-A1- 2001 018 077
- US-B1- 6 713 116
- DATABASE WPI Section Ch, Week 200346 Derwent Publications Ltd., London, GB; Class B03, AN 2003-485823 XP002306600 & JP 2002 335926 A (IKKO KAGAKU KK) 26 November 2002 (2002-11-26)
- DATABASE WPI Section Ch, Week 200215 Derwent Publications Ltd., London, GB; Class D13, AN 2002-111697 XP002306601 & KR 2001 079 226 A (SAM BO NATURAL CO LTD) 22 August 2001 (2001-08-22)
- DATABASE WPI Section Ch, Week 200347 Derwent Publications Ltd., London, GB; Class B04, AN 2003-498138 XP002306602 & JP 2003 055234 A (HANA HEALTH KK) 26 February 2003 (2003-02-26)
- DATABASE WPI Section Ch, Week 200411 Derwent Publications Ltd., London, GB; Class B04, AN 2004-102946 XP002306603 & JP 2004 010605 A (TANAKA Y) 15 January 2004 (2004-01-15)
- DATABASE WPI Section Ch, Week 200239 Derwent Publications Ltd., London, GB; Class D13, AN 2002-353022 XP002306604 & CN 1 336 142 A (TANG Z) 20 February 2002 (2002-02-20)
- PATENT ABSTRACTS OF JAPAN vol. 2003, no. 12, 5 December 2003 (2003-12-05) & JP 2004 075692 A (ITSUKOU KAGAKU KK), 11 March 2004 (2004-03-11)
- PATENT ABSTRACTS OF JAPAN vol. 2000, no. 09, 13 October 2000 (2000-10-13) & JP 2000 157183 A (NAKAJIMA FUMIO), 13 June 2000 (2000-06-13)
- DATABASE WPI Section Ch, Week 200146 Derwent Publications Ltd., London, GB; Class B05, AN 2001-430654 XP002306605 & KR 2001 001 290 A (PACIFIC IND CO) 5 January 2001 (2001-01-05)
- GARLEB K A ET AL: "EFFECT OF FRUCTOOLIGOSACCHARIDE CONTAINING ENTERAL FORMULAS ON SUBJECTIVE TOLERANCE FACTORS, SERUM CHEMISTRY PROFILES, AND FAECAL BIFIDOBACTERIA IN HEALTHY ADULT MALE SUBJECTS" MICROBIAL ECOLOGY IN HEALTH & DISEASE, CHICHESTER, GB, vol. 9, no. 6, 1996, pages 279-285, XP008023764

## Description

### Field of the invention

The present invention is related to the area of alimentation and concerns the use of mixtures comprising said actives and prebiotics for improving the stimulation of the growth of healthy bacteria.

### Background of the invention

Probiotics contain live bacteria and represent an important part of the complex world of foods that are good for health. Its the bacteria and the metabolites which they produce that give these products their health promoting properties. The best known example of a probiotic is yoghurt. The experimental data for yoghurt is still not as conclusive as one would like, however, human studies related to the consumption of dietary milk products show increased milk digestibility, quicker recovery from certain types of diarrhoea, enhanced immune function, relation in certain cancers, and possible lowering of blood cholesterol levels.

Bacteria found in products like yoghurt, kefir or fermented vegetables usually aren't found in the human intestine. In fact, the intestinal environment is often a hostile one for these foreign bacteria. Because of this, bacteria eaten in probiotic products don't colonise the intestine but are flushed through and eliminated from the body.

The bacteria living in the intestine make up a very large and very diverse population. The numbers of each kind of bacteria change depending on age, diet, health status, and use of drugs and supplements. The effects are linked to the ability of the bacteria to adhere to the intestinal wall and use the semi-digested food that it passing through the intestines. It is not surprising to found that the bacterial population in the intestines of vegetarians is much different compared to that of meat eaters. Because some bacteria have specific nutrient requirements it has been proposed that adding these particular foods or nutrients to the diet could be a way of increasing the numbers of specific bacteria. Such additives are called "prebiotics".

Thus, to be effective, prebiotics mush escape digestion in the upper gastrointestinal tract and be used by a limited number of the micro-organisms comprising the colonic microflora. Typical examples for well-known prebiotics are oligosaccharides, e.g. in 1995 Gibson et al found that oligofructose and inulin, when fed to humans, selectively stimulated the growth of bifidobacteria without influencing the numbers of lactobacillus. Since prebiotics mainly stimulate the growth of bifidobacteria, for which reason the also are referred to as bifidogenetic factors.

Although various types of prebiotics are known from the literature and can be found in the market there is still an increasing need for more active alternatives or additives which support the various activities of existing products in synergistic manner. Therefore, the object of the present invention has been to provide a new system of prebiotic compounds which shows a synergistic stimulation of the growth of healthy bacteria, preferably bifido and lactic bacteria both.

US 5 071 653 discloses the use of green tea extract for promoting the growth of bifidobacterium in the human intestine.

Garleb et al. (Microbial ecology in health and disease, Chichester, GB, vol.9, n° 6, p. 279 - 285, 1996) discloses the use of fructooligosaccharides for promoting the growth of bacteria in the human intestine.

### Detailed description of the invention

The present invention refers to the use of mixtures, comprising
(a) prebiotics and
(b) polyphenols or plant extracts rich in polyphenols, for stimulating the growth of bacteria selected from the group consisting of *Bifidobacterium breve, Bifidobacterium infantis, Bifidobacterium longum* and *Bifidobacterium adolescentis* on one band, and *Lactobacillus bulgaricus, Lactobacillus acidophilus, Lactobacillus casei, Lactobacillus plantarum, Streptococcus faccium, and Streptococcus thermophilus*.

Surprisingly it has been observed that mixtures of various types of polyphenols preferably of plant origin and prebiotics show a synergistic behaviour with respect to stimulation of growth of the bacteria as mentioned above.

### Prebiotics

Prebiotics are defined as non-digestible food ingredients that may beneficially affect the host be selectively stimulating the growth and/or the activity of a limited number of bacteria in the colon. The following describes the various oligosaccharides which can be taken into account as suitable prebiotics (component a) :

### • Fructooligosaccharides

Fructooligosaccharides or FOS typically refer to short-chain oligosaccharides comprised of D-fructose and D-glucose, containing from three to five monosaccharide units. FOS, also called neosugar and short-chain FOS, are produced on a commercial scale from sucrose using a fungal fructosyltransferase enzyme. FOS are resistant to digestion in the upper gastrointestinal tract. They act to stimulate the growth of *Bifidobacterium* species in the large intestine. FOS are marketed in the United States in combination with probiotic bacteria and in some functional food products.

### • Inulins

Inulins refer to a group of naturally-occurring fructose-containing oligosaccharides. Inulins belong to a class of carbohydrates known as fructans. They are derived from the roots of chicory *(Cichorium intybus)* and Jerusalem artichokes. Inulins are mainly comprised of fructose units and typically have a terminal glucose. The bond between fructose units in inulins is a beta-(2-1) glycosidic linkage. The average degree of polymerisation of inulins marketed as nutritional supplements is 10 to 12. Inulins stimulate the growth of *Bifidobacterium* species in the large intestine.

### • Isomaltooligosaccharides

Isomaltooligosaccharides comprise a mixture of alpha-D-linked glucose oligomers, including isomaltose, panose, isomaltotetraose, isomaltopentaose, nigerose, kojibiose, isopanose and higher branched oligosaccharides. Isomaltooligosaccharides are produced by various enzymatic processes. They act to stimulate the growth *of Bifidobacterium* species and *Lactobacillus* species in the large intestine. Isomalto oligosaccharides are marketed in Japan as dietary supplements and in functional foods. They are being developed in the United States for similar uses.

### • Lactilol

Lactilol is a disaccharide analogue of lactulose. Its pharmaceutical use is in the treatment of constipation and hepatic encephalopathy. Lactilol is also used in Japan as a prebiotic. It is resistant to digestion in the upper gastrointestinal tract and it is fermented by a limited number of colonic bacteria, resulting in an increase in the biomass of bifidobacteria and lactobacilli in the colon. Lactilol is known chemically as 4-0-(beta-D-galactopyranosyl)-D-glucitol. Lactilol is not approved for the treatment of hepatic encephalopathy or constipation in the U.S., and its use as a prebiotic is considered experimental. Lactilol is used in Europe as a food sweetener.

### • Lactosucrose

Lactosucrose is a trisaccharide comprised of D-galactose, D-glucose and D-fructose. Lactosucrose is produced enzymatically by the enzymatic transfer of the galactosyl residue in lactose to sucrose. Lactosucrose is resistant to digestion in the stomach and small intestine. It is selectively utilized by intestinal *Bifidobacterium* species resulting in significant induction of growth of these bacteria in the colon. Therefore, under physiological conditions, lactosucrose acts on the intestinal microflora as a growth factor for *Bifidobacterium* species. Lactosucrose is also known as 4G-beta-D-galactosylsucrose. It is widely used in Japan as a dietary supplement and in functional foods, including yoghurt. Lactosucrose is being developed in the United States for similar uses.

### • Lactulose

Lactulose is a semi-synthetic disaccharide comprised of the sugars D-lactose and D-fructose. The sugars are joined by a beta-glycosidic linkage, making it resistant to hydrolysis by human digestive enzymes. Lactulose is, however, fermented by a limited number of colonic bacteria. This can lead to changes in the colonic ecosystem in favour of bacteria, such as lactobacilli and bifidobacteria, which may confer some health benefits. Lactulose is a prescription drug in the United States for the treatment of constipation and hepatic encephalopathy. It is marketed in Japan for use as a dietary supplement and in functional foods. Its use in the United States as a prebiotic substance is still experimental.

### • Pyrodextrins

Pyrodextrins comprise a mixture of glucose-containing oligosaccharides that is derived from the hydrolysis of starch. Pyrodextrins have been found to promote the proliferation of *Bifidobacterium* species in the large intestine. They are resistant to digestion in the upper gastrointestinal tract. Pyrodextrins are being developed for the nutritional supplement market place.

### • Soy oligosaccharides

Soy oligosaccharides refer to oligosaccharides found in soybeans and also in other beans and peas. The two principal soy oligosaccharides are the trisaccharide raffinose and the tetrasaccharide stachyose. Raffinose comprises one molecule each of D-galactose, D-glucose and D-fructose. Stachyose consists of two molecules of D-galactose, one molecule of D-glucose and one molecule of D-fructose. Soy oligosaccharides act to stimulate the growth of *Bifidobacterium* species in the large intestine. They are marketed in Japan as dietary supplements and in functional foods. They are being developed in the United States for similar uses.

### • Transgalactooligosaccharides

Transgalactooligosaccharides (TOS) are a mixture of oligosaccharides consisting of D-glucose and D-galactose. TOS are produced from D-lactose via the action of the enzyme beta-galactosidase obtained from *Aspergillus oryzae.* TOS are resistant to digestion in the upper gastrointestinal tract and stimulate the growth of bifidobacteria in the large intestine. TOS are marketed in Japan and Europe as dietary supplements and are used in functional foods. They are being developed for similar use in the United States.

### • Xylooligosaccharides

Xylooligosaccharides are comprised of oligosaccharides containing beta (1→ 4) linked xylose residues. The degree of polymerisation of xylooligosaccharides is from two to four. Xylo oligosaccharides are obtained by enzymatic hydrolysis of the polysaccharide xylan. They are marketed in Japan as prebiotics and are being developed for similar use in the United States.

### • Beta Glucans

Suitable beta-glucans include those originating from plants including cereals such as oats and barley, fungi, yeast, and bacteria. In addition, microbial cell wall preparations and whole cells rich in beta glucans are also suitable sources for beta glucan preparations useful for the present invention. Monomer residues in glucans can have 1-3 and 1-4, or 1-3 and 1-6 linkages (that is the monomer units are joined through 1,3, 1,4 or 1,6 bonds) and the percent of each type can vary. Preferably, beta glucans derived from yeast, particularly from *Saccharomyces,* preferably *Saccharomyces cerevisiae,* are used for the present invention. It will be appreciated, however, that other beta glucans would also be suitable.

### Polyphenols and plant extracts rich in polyphenols

Preferably, the polyphenols of the mixtures implemented in the present invention are selected from the group consisting of (iso)flavons, (iso)flavonols, (iso)flavonones, (iso)flavonoids, catechins, ginkgolides A, B, C bilobalides, oliogoprocyanidins and their glycosides.

In the following examples for polyphenols and plant extracts which are rich in said polyphenols are given in order to illustrate the invention, but not to limit the invention to the selected species:

### • Ginkgo biloba

The active ingredients of the extract are flavonoid glycosides, which among others contain (iso)quercitin glycosides, kaempferol, kaempferol-3-rhamnosides, isorhamnetin, luteolin glycosides, sitosterol glycosides and predominantly hexacyclic terpene lactones, consisting of ginkgolides A, B, C, J, M and bilobalides. 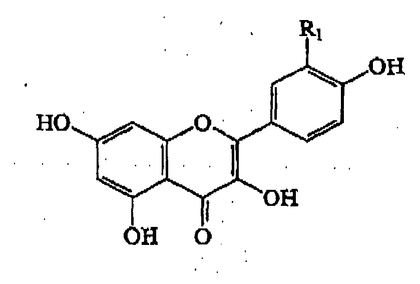 Isorhamnetin (R¹ = H), Kaempferol (R¹ = OH), Ginkgolide A (R¹ = OMe)

### • Oleacea europensis

The main constituent of the leaves of the olive tree (*Oleacea europensis*) is the antioxidant oleuropein, which is also the main source for hydroxytyrosol.

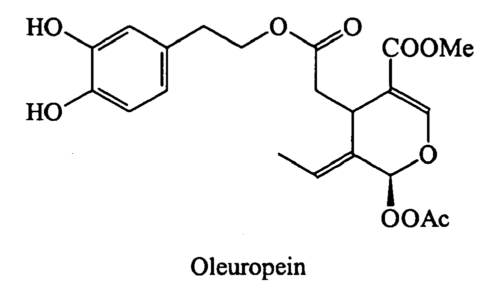

### • Camellia sinensis

Polyphenols of the catechin and flavonoid type, so-called "tea-tannins" represent the main active principles of extracts of Green Tea (*Camellia sinensis*) :

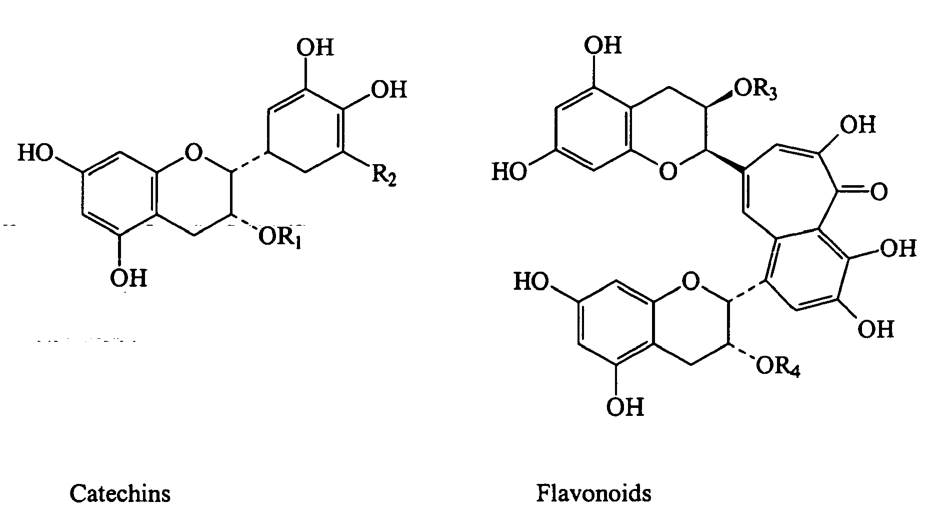

| | R1 | R2 | R3 | R4 |
|---|---|---|---|---|
| (-)-Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theaflavin | | | H | H |
| Theaflavin monogallate A | | | Galloyl | H |
| Theaflavin monogallat B | | | H | Galloyl |
| Theaflavin digallate | | | Galloyl | Galloyl |

### • Trifolium pratense

The main active principles of red clover (*Triflolium pratense*) are isoflavones, like e.g. daidzein, genestein, formononentin and biochanin as well as their glucosides like ononin or sissostrin:

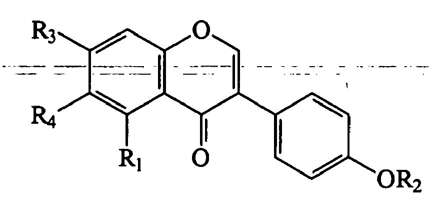

| **Isoflavonglucosides** | **R₁** | **R₂** | **R₃** | **R₄** |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### • Litchi sinensis

Extracts of pericarps from Litchi (*Litchi sinensis*) are well known for its high content of flavone derivatives like e.g. 2-phenyl-4H-1-benzopyrans, flavans, flavan-3-ols (catechins, catechin oligomers), flavan-3,4-diols (leucoanthocyanides), flavones, flavonols and flavonones. The main component, however, represent condensed tannins, so-called procyanodols (OPC). These compounds comprise 2 to 8 monomers of the catechin or epicatechin-type, like e.g. procyanidins, proanthocynidins, procyanidol, oligoprocyanidins, leucoanthocyanidins, leucodelphinins, leucocyanins and anthocyanogens. OPC, mainly the preferred proanthocyanidin A2 (OPC A2) behave like vitamin P, especially with respect to MMP inhibition.

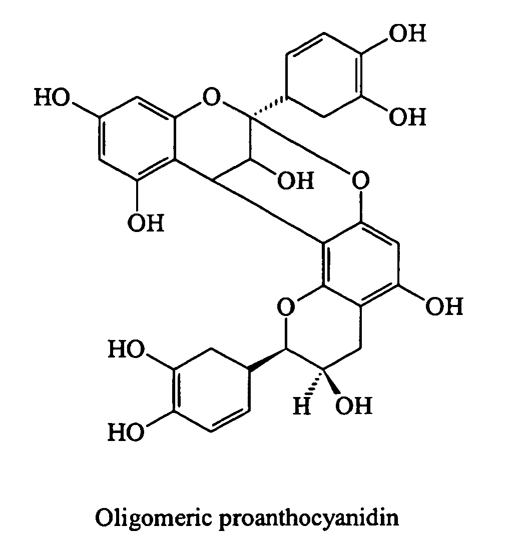

### • Passiflora incarnata

Extracts of passion flower *(Passiflora incarnata)* are rich in flavones of the apigenin and luteolin-type and their C-glycosides: 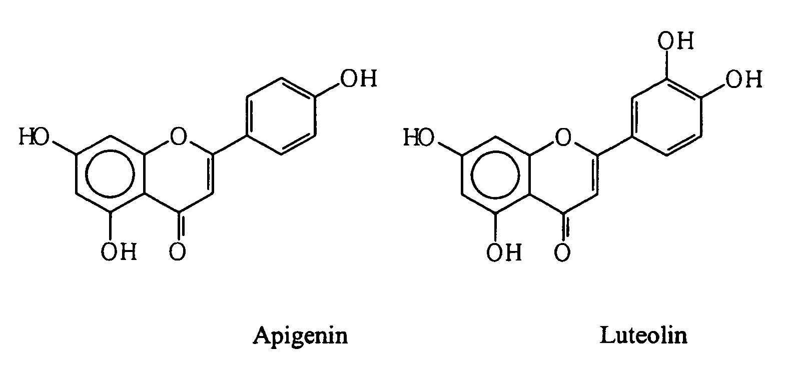 In addition they comprise 2"-B-D-glucosides, schaftosides and iso-schaftosides, isovitexin, isoorientin, vicenin-2, incenin-2, daponanin and trace elements like calcium, phosphorans und iron.

### • Medicago sativa

Extracts of Alfalfa (*Medicago sativa*) are rich in isoflavones like e.g. daidzein, genestein, formononetin, biochanin A und tricin :

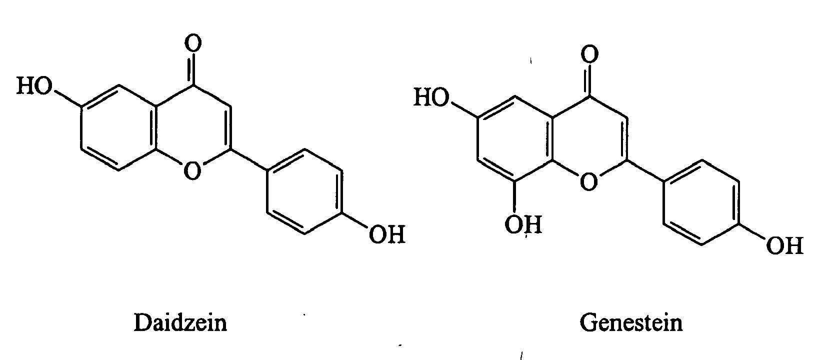

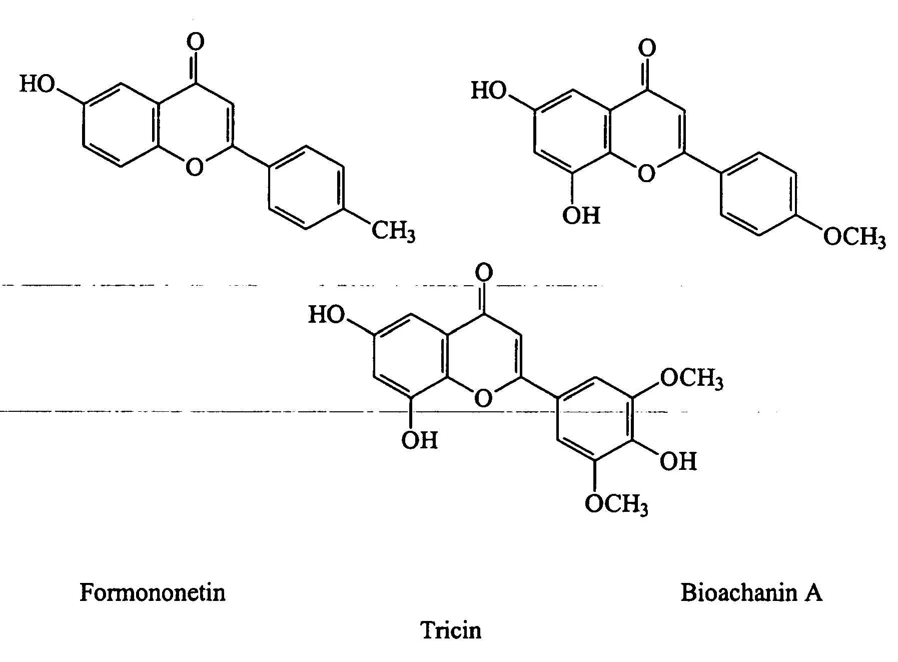

According to an advantageous embodiment of the present invention, the above-mentioned mixtures may comprise the prebiotics and the polyphenols in a weight ratio of 99 to 1 to 50 : 50 and more particularly 95 : 10 to 75 : 25. The highest synergistic effects, however, are observed at ratios of 92 ; 8 to 80 : 20. In general, the mixtures can be used in a concentration of up to about 10, particularly 0.5 to 8 and more particularly 1 to 2 % b.w. - calculated on the probiotic micro-organisms being present in the final food composition. One percent, however, has been found to be particularly suitable.

In a special embodiment of the present invention said mixtures are macro- or micro-encapsulated. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, $s microparticles, may be additionally coated with film-forming polymers, The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl *cellulose*), *Kobo G*/*ycospheres* (modified starch, fatty acid esters, phospholipids), *Softspheres* (modified agar agar), *Kuhs Probiol Nanospheres* (phospholipide) and *Primaspheres* or *Primasponges* (chitosan, anionic polymers).

A further object of the present invention relates to the above-mentioned use, wherein said mixtures are used as food compositions.

In the present invention, the above-mentioned mixtures are used
for stimulating the growth of healthy bacteria, for example in the stomach (if applied orally) or on skin (if administered topical).

### Examples

### Examples 1 to 10, Comparative Examples C1 to C18

The stimulation of growth of micro-organisms has been studied by enumerating *bifidobacterium* and *lactobacilli* in vitro in the presence of various test substances:
- Extract A :: Extract of *Trifolium pratense* (Red clover)
- Extract B :: Extract of *Camellia sinensis* (Green tea)
- Extract C :: Extract of *Oleacea europensis* (Olive tree)
- Extract D :: Extract of *Ginkgo biloba* (Ginkgo tree)

All extracts show an active content of about 20 % b.w. and are commercially available from Cognis Deutschland GmbH & Co. KG.

Aliquots (1 mL) of human faecal homogenates (10 g per 100 mL diluent) were added to diluted WC broth (diluted 50:50 with 0.05M phosphate buffer) to which were added the test mixtures and a lactobacillus or bifidobacterium strain. For each of the combinations, parallel tubes were prepared with one set being inoculated with *Bifidobacterium spp* or *Lactobacillus spp*. All mixtures were then incubated for up to 24 hours and bacterial numbers enumerated. The results are presented in Tables 1 and 2 (amount of extract calculated on active content):

**Table 1**

| **Effect of 1 % prebiotic, plant extracts and prebiotic/plant extract mixture on *Bifidobacterium*** | | | | | | | | | | | | | | |
|---|---|---|---|---|---|---|---|---|---|---|---|---|---|---|
| | **0** | **C1** | **C2** | **C3** | **C4** | **C5** | **C6** | **C7** | **C8** | **1** | **2** | **3** | **4** | **5** |
| Inulin | - | 1.0 | - | - | - | - | - | - | - | 0.8 | 0.9 | - | - | - |
| Lactosucrose | - | - | 1.0 | - | - | - | - | - | - | - | - | 0.9 | - | - |
| Lactolin | - | - | - | 1.0 | - | - | - | - | - | - | - | - | 0.9 | - |
| Betaglucan | - | - | - | - | 1.0 | - | - | - | - | - | - | - | - | 0.9 |
| Extract A | - | - | - | - | - | 1.0 | - | - | - | 0.2 | - | - | - | - |
| Extract B | - | - | - | - | - | - | 1.0 | - | - | - | 0.1 | 0.1 | - | - |
| Extract C | - | - | - | - | - | - | - | 1.0 | - | - | - | - | 0.1 | - |
| Extract D | - | - | - | - | - | - | - | - | 1.0 | - | - | - | - | 0.1 |
| Bacterial numbers (CFU/ml) | 1.0 x 10⁶ | 1.5 x 10⁷ | 1.1 x 10⁷ | 1.6 x 10⁷ | 1.2 x 10⁷ | 3.3 x 10⁶ | 2.3 x 10⁶ | 2.7 x 10⁶ | 3.4 x 10⁶ | 4.1 x 10⁷ | 4.3 x 10⁷ | 4.0 x 10⁷ | 4.0 x 10⁷ | 4.4 x 10⁷ |

Starting from a control of 1.0 10⁶ CFU/ml (0) the addition of 1 % b.w. of various prebiotics (Comparative Examples C1-C4) increases the CFU by a factor of 10, while the addition of the plant extracts does only have a weak effect on the stimulation of cell growth (Comparative Examples C5-C8). Adding however mixture of prebiotics and plant extracts to the samples, the CFU numbers were multiplied by a factor of about 40 (Inventive Examples 1 to 5). The highest synergistic effect can be seen at a relation prebiotic : polyphenols of about 90 : 10.

**Table 2**

| **Effect of 1 % prebiotic, plant extract and prebiotic/plant extract mixture on *Lactobacterium*** | | | | | | | | | | | | | | |
|---|---|---|---|---|---|---|---|---|---|---|---|---|---|---|
| | **0** | **C9** | **C10** | **C11** | **C12** | **C13** | **C14** | **C15** | **C16** | **6** | **7** | **8** | **9** | **10** |
| Inulin | - | 1.0 | - | - | - | - | - | - | - | 0.8 | 0.9 | - | - | - |
| Lactosucrose | - | - | 1.0 | - | - | - | - | - | - | - | - | 0.9 | - | - |
| Lactolin | - | - | - | 1.0 | - | - | - | - | - | - | - | - | 0.9 | - |
| Betaglucan | - | - | - | - | 1.0 | - | - | - | - | - | - | - | - | 0.9 |
| Extract A | - | - | - | - | - | 1.0 | - | - | - | 0.2 | - | - | - | - |
| Extract B | - | - | - | - | - | - | 1.0 | - | - | - | 0.1 | 0.1 | - | - |
| Extract C | - | - | - | - | - | - | - | 1.0 | - | - | - | - | 0.1 | - |
| Extract D | - | - | - | - | - | - | - | - | 1.0 | - | - | - | - | 0.1 |
| Bacterial numbers (CFU/ml) | 2.8 x 10⁵ | 1.4 x 10⁶ | 1.1 x 10⁶ | 1.5 x 10⁶ | 1.1 x 10⁶ | 4.2 x 10⁵ | 4.3 x 10⁵ | 4.4 x 10⁵ | 4.5 x 10⁵ | 6.5 x 10⁵ | 6.3 x 10⁶ | 6.7 x 10⁶ | 6.6 x 10⁶ | 6.9 x 10⁶ |

Starting from a control of 2.8 10⁵ CFU/ml (0) the addition of 1 % b.w. of various prebiotics (Comparative Examples C9-C12) increases the CFU by a factor of 4, while the addition of the plant extracts does only have a weak effect on the stimulation of cell growth (Comparative Examples C13-C16). Adding however, mixture of prebiotics and plant extracts to the samples, the CFU numbers were multiplied by a factor of about 15 (Inventive Examples 6 to 10). The highest synergistic effect can be seen again at a relation prebiotic : polyphenols of about 90 : 10.

### Example 11

### Yoghurt composition

Soy milk is added to 15-75 parts by volume of cow milk to make 100 parts of the mixture. The mixture is then pasteurised at about 90 °C. for 15 seconds and then cooled. The cooled, pasteurised mixtures are then inoculated with 3 to 5 percent by volume of a yoghurt culture having 1:1 ratio of *Lactobacillus bulgaricus* and *Bifidobacterium adolescentis*. The incubation is carried out at about 42 °C. In about 2 hours thickening will occur. The fermentation is carried out for about 5.5 hours. The yoghurt compositions thus obtained is treated with 1 % - calculated on the amount of micro-organisms being present - of a 9:1 mixture of inulin and an extract of Green Tea. The products firm consistency and have a flavour like or substantially indistinguishable from that of a corresponding yoghurt composition using 100 percent of fresh cow milk. A small amount of citric acid can be added to the fermentation mixture to enhance the flavour of the final yoghurt composition. A suitable amount of citric acid is 0.5 percent based on the weight of the composition.

## Claims

1. Use of mixtures, comprising
(i) Prebiotics and
(ii) Polyphenols or plant extracts rich in polyphenols
for stimulating the growth of bacteria selected from the group consisting of *Bifidobacterium breve, Bifidobacterium infantis, Bifidobacterium longum, Bifidobacterium adolescentis, Lactobacillus bulgaricus, Lactobacillus acidophilus, Lactobacillus casei, Lactobacillus plantarum, Streptococcus faecium,* and *Streptococcus thermophilus.*

2. Use according to Claim 1, **characterised in that** said prebiotics (component i) are selected from the group consisting of fructooligosaccharides, inulins, isomaltooligosaccharides, lactilol, lactosucrose, Lactulose, Pyrodextrins, soy oligosaccharides, transgalactooligosaccharides, xylooligosaccharides and beta glucans.

3. Use according to claims 1 and/or 2, **characterised in that** said polyphenols (component ii) are selected from the group consisting of (iso)flavons, (iso)flavonols, (iso)flavonones, iso)flavonoids, catechins, ginkgolides A, B, C, bilobalides, oliogoprocyanidins and their glycosides.

4. Use according to any of the Claims 1 to 3, **characterised in that** said polyphenols (component ii) are selected from the group consisting of (iso)quercitin, kaempferol, isorhamnetin, luteolin, oleuropein, hydroxytyrosol, (epi)catechin(gallate), (epi)gallocatechin(gallate), theaflavin(gallate), daidzein, genestein, formononentin, biochanin A, tricin, proanthocyanidin A2, apigenin, luteolin and their glycosides.

5. Use according to any of the Claims 1 to 4, **characterised in that** said plant extracts (component ii) represent extracts of *Ginkgo biloba, Camellia sinensis, Trifolium pratense, Oleacea europensis, Litchi sinensis, Passiflora incarnata, Medicago sativa* and their mixtures.

6. Use according to any of the Claims 1 to 5, **characterised in that** said mixtures comprise components (i) and (ii) in weight ratios of from 99:1 to 50:50.

7. Use according to any of the Claims 1 to 6, **characterised in that** said mixtures are used in amounts of up to 10 % b.w. calculated on the micro-organisms being present in the final food composition.

8. Use according to any of the Claims 1 to 7, **characterised in that** said mixtures of compounds (i) and (ii) are macro- or micro-encapsulated.

9. Use according to any of the Claims 1 to 8, **characterised in that** said compositions represent food compositions.

## Patentansprüche

1. Verwendung von Gemischen, umfassend:
(i) Präbiotika und
(ii) Polyphenole oder an Polyphenolen reiche Pflanzenextrakte
zum Stimulieren des Wachstums von Bakterien, die aus der Gruppe ausgewählt sind, die aus Bifidobacterium breve, Bifidobacterium infantis, Bifidobacterium longum, Bifidobacterium adolescentis, Lactobacillus bulgaricus, Lactobacillus acidophilus, Lactobaciullus casei, Lactobacillus plantarum, Streptococcus faecium und Streptococcus thermophilus besteht.

2. Verwendung nach Anspruch 1, **dadurch gekennzeichnet, daß** die Präbiotika (Komponente i) aus der Gruppe ausgewählt sind, die aus Fructooligosacchariden, Inulinen, Isomaltooligosacchariden, Lactilol, Lactosucrose, Lactulose, Pyrodextrinen, Sojaoligosacchariden, Transgalactooligosacchariden, Xylooligosacchariden und β-Glucanen besteht.

3. Verwendung nach Anspruch 1 und/oder 2, **dadurch gekennzeichnet, daß** die Polyphenole (Komponente ii) aus der Gruppe ausgewählt sind, die aus (Iso)flavonen, (Iso)flavonolen, (Iso)flavononen, (Iso)flavonoiden, Catechinen, den Ginkgoliden A, B, C, Bilobaliden, Oligoprocyanidinen und deren Glycosiden besteht.

4. Verwendung nach einem der Ansprüche 1 bis 3, **dadurch gekennzeichnet, daß** die Polyphenole (Komponente ii) aus der Gruppe ausgewählt sind, die aus (Iso)quercitin, Kämpferol, Isorhamnetin, Luteolin, Oleuropein, Hydroxytyrosol, (Epi)catechin(gallat), (Epi)gallocatechin(gallat), Theaflavin(gallat), Daidzein, Genestein, Formononentin, Biochanin A, Tricin, Proanthocyanidin A2, Apigenin, Luteolin und deren Glycosiden besteht.

5. Verwendung nach einem der Ansprüche 1 bis 4, **dadurch gekennzeichnet, daß** die Pflanzenextrakte (Komponente ii) Extrakte von Ginkgo biloba, Camellia sinensis, Trifolium pratense, Oleacea europensis, Litchi sinensis, Passiflora incarnata, Medicago sativa und deren Gemische darstellen.

6. Verwendung nach einem der Ansprüche 1 bis 5, **dadurch gekennzeichnet, daß** die Gemische die Komponenten (i) und (ii) in Gewichtsverhältnissen von 99:1 bis 50:50 umfassend.

7. Verwendung nach einem der Ansprüche 1 bis 6, **dadurch gekennzeichnet, daß** die Gemische in Mengen von bis zu 10 Gew.-% verwendet werden, und zwar auf die Mikroorganismen bezogen berechnet, die in der abschließenden Nahrungsmittelzusammensetzung vorliegen.

8. Verwendung nach einem der Ansprüche 1 bis 7, **dadurch gekennzeichnet, daß** die Gemische der Verbindungen (i) und (ii) makro- oder mikroverkapselt sind.

9. Verwendung nach einem der Ansprüche 1 bis 8, **dadurch gekennzeichnet, daß** die Zusammensetzungen Nahrungsmittelzusammensetzungen darstellen.

## Revendications

1. Utilisation de mélanges, comprenant
(i) des prébiotiques et
(ii) des polyphénols ou extraits végétaux riches en polyphénols
pour stimuler la croissance de bactéries choisies dans le groupe constitué par *Bifidobacterium breve, Bifidobacterium infantis, Bifidobacterium longum, Bifidobacterium adolescentis, Lactobacillus bulgaricus, Lactobacillus acidophilus, Lactobacillus casei, Lactobacillus plantarum, Streptococcus faecium, et Streptococcus thermophilus.*

2. Utilisation selon la revendication 1, **caractérisée en ce que** lesdits prébiotiques (composant i) sont choisis dans le groupe constitué par les fructooligosaccharides, les inulines, les isomaltooligosaccharides, le lactilol, le lactosaccharose, le lactulose, les pyrodextrines, les oligosaccharides de soja, les transgalactooligosaccharides, les xylooligosaccharides et les bêta glucanes.

3. Utilisation selon les revendications 1 et/ou 2, **caractérisée en ce que** lesdits polyphénols (composant ii) sont choisis dans le groupe constitué par les (iso)flavones, les (iso)flavonols, les (iso)flavonones, les (iso)flavonoïdes, les catéchines, les ginkgolides A, B, C, les bilobalides, les oligoprocyanidines et leurs glycosides.

4. Utilisation selon l'une des revendications 1 à 3, **caractérisée en ce que** lesdits polyphénols (composant ii) sont choisis dans le groupe constitué par l'(iso)quercitrine, le kaempférol, l'isorhamnétine, la lutéoline, l'oleuropéine, l'hydroxytyrosol, le (gallate) d'(épi)catéchine, le (gallate) d'(épi)gallocatéchine, le (gallate)de théaflavine, la daidzéine, le génestéine, la formononentine, la biochanine A, la tricine, la proanthocyanidine A2, l'apigénine, la lutéoline et leurs glycosides.

5. Utilisation selon l'une quelconque des revendications 1 à 4, **caractérisée en ce que** lesdits extraits végétaux (composant ii) représentent des extraits de *Ginkgo biloba, Camellia sinensis, Trifolium pratense, Oleacea europensis, Litchi sinensis, Passiflora incarnata, Medicago saliva* et leurs mélanges.

6. Utilisation selon l'une quelconque des revendications 1 à 5, **caractérisée en ce que** lesdits mélanges comprennent les composants (i) et (ii) à des rapports en poids allant de 99:1 à 50:50.

7. Utilisation selon l'une quelconque des revendications 1 à 6, **caractérisée en ce que** lesdits mélanges sont utilisés à des quantités allant jusqu'à 10 % en poids, calculées par rapport aux micro-organismes présents dans la composition alimentaire finale.

8. Utilisation selon l'une quelconque des revendications 1 à 7, **caractérisée en ce que** lesdits mélanges de composés (i) et (ii) sont macro- ou micro-encapsulés.

9. Utilisation selon l'une quelconque des revendications 1 à 8, **caractérisée en ce que** lesdites compositions représentent des compositions alimentaires.
